# EUROPEAN PATENT SPECIFICATION

(11) **EP 4 344 415 B1**
(45) Date of publication and mention of the grant of the patent: **03.06.2026**
(21) Application number: 22757666.7
(22) Date of filing: 04.08.2022
(51) Int. Cl.: A61N 1/36, A61K 39/00, A61N 1/04

(54) **ELECTRODE ASSEMBLY FOR APPLYING TUMOR TREATING FIELDS THAT INCLUDE A SHEET OF GRAPHITE**
ELEKTRODENANORDNUNG ZUR ANWENDUNG VON TUMORBEHANDLUNGSFELDERN (TTFIELDS) MIT EINER GRAPHITFOLIE
ENSEMBLE ÉLECTRODE POUR L'APPLICATION DE CHAMPS DE TRAITEMENT DE TUMEURS (TTCHAMPS) COMPRENANT UNE FEUILLE DE GRAPHITE

(30) Priority: 06.08.2021 US 202163230438 P; 04.11.2021 US 202163275841 P; 04.11.2021 US 202163275843 P
(43) Date of publication of application: 03.04.2024
(73) Proprietor: Novocure GmbH, 6340 Baar (CH)
(72) Inventor: WASSERMAN, Yoram, 31905 Haifa (IL); OBUCHOVSKY, Stas, 31905 Haifa (IL); KUPLENNIK, Nataliya, 31905 Haifa (IL); SHAPIRO, David, 31905 Haifa (IL)
(74) Representative: Carpmaels & Ransford LLP
(86) International application number: PCT/IB2022/057234
(87) International publication number: WO 2023/012708

(56) References cited:
- CN-A- 108 568 029
- US-A1- 2006 276 858
- US-A1- 2021 138 233
- US-A1- 2021 162 228

## Description

### BACKGROUND

Tumor Treating Fields (TTFields) therapy is a proven approach for treating tumors using alternating electric fields at frequencies between 50 kHz and 1 MHz, such as, for example, 100-500 kHz. The alternating electric fields are induced by electrode assemblies (e.g., arrays of capacitively coupled electrodes, also called transducer arrays) placed on opposite sides of the subject's body. When an AC voltage is applied between opposing electrode assemblies, an AC current is coupled through the electrode assemblies and into the subject's body. And higher currents are strongly correlated with higher efficacy of treatment.

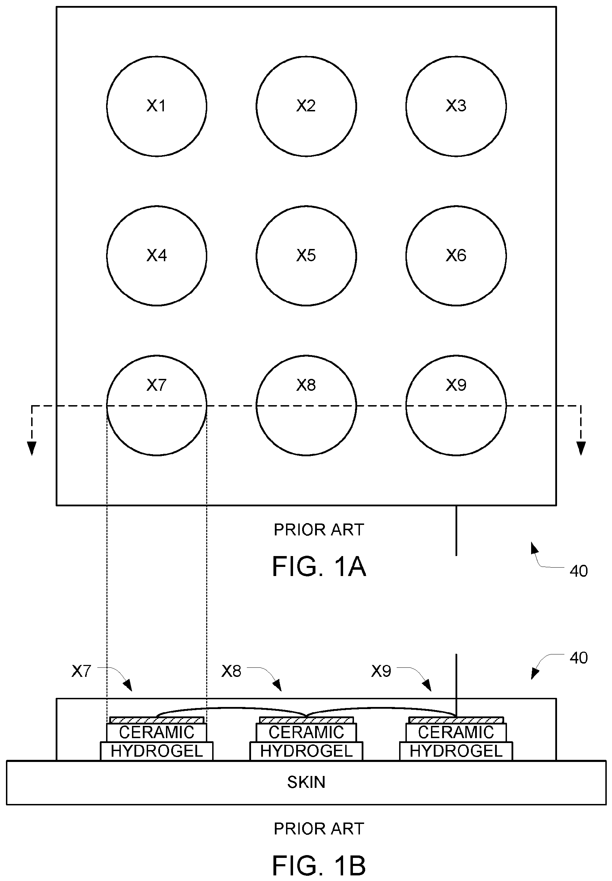

FIG. 1A is a schematic representation of a prior art electrode assembly 40 including nine prior art electrode elements, labeled X1-X9. FIG. 1B is a cross sectional schematic view of electrode elements X7-X9 of the electrode assembly 40, taken along the dashed line in FIG. 1A.

As shown in FIG. 1B, electrode element X7 (taken as exemplary) includes a metal layer (shown with diagonal hatching) and a ceramic (dielectric) layer. A respective layer of electrically conductive hydrogel is provided between each ceramic layer and the subject's skin, to ensure good electrical contact of the electrode elements with the body. An AC voltage from an AC voltage generator (not shown) is applied to the metal layers of electrode elements in opposing electrode assemblies to generate the TTFields in the subject's body.

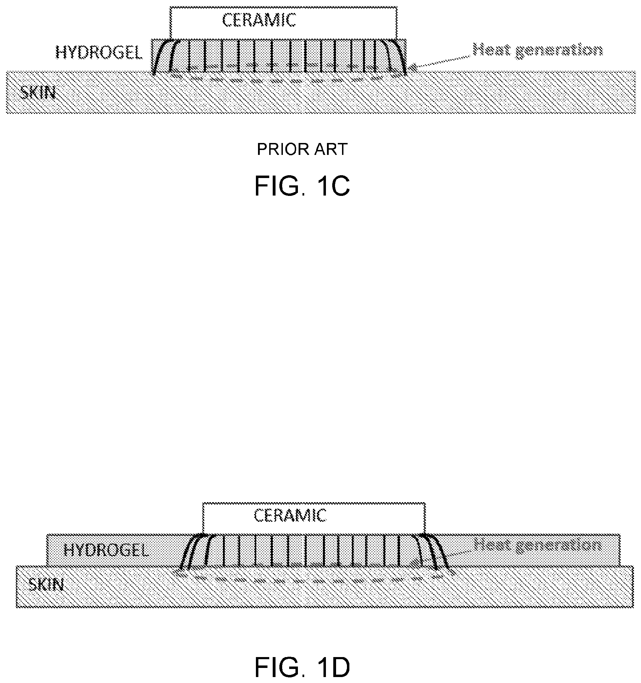

During use, the hydrogel and the skin under the electrode elements heat up, and safety considerations require that the skin temperature remain below a safety threshold (e.g., 41° C). Because the vast majority of the heat appears immediately below the electrode elements X1-X9 (as shown in FIG. 1C), the prior art electrode assembly has hot spots immediately below the electrode elements, and cooler regions positioned between the electrode elements. And those hot spots limit the amount of current that can be delivered through the prior art electrode assemblies.

US-A-2006/0276858 discloses the provision of a conductive layer between a dielectric and a patient's body to improve the electrical contact with the patient's body. Examples of suitable materials for the conductive layer include conductive gels and carbon (graphite) powders, which maybe imbedded in a suitable cream (e.g. a cosmetic base with an electrolyte).

### SUMMARY OF THE INVENTION

One aspect of the invention is directed to an apparatus for applying an alternating electric field to a subject's body according to claim 1.

Embodiments of the invention are disclosed in the dependent claims.

### BRIEF DESCRIPTION OF THE DRAWINGS

FIG. 1A is a schematic representation of a prior art electrode assembly.
FIG. 1B is a cross sectional view of electrode elements of the prior art electrode assembly, taken along the dashed line in FIG. 1A.
FIG. 1C is a cross sectional view showing the heat generation properties of a prior art electrode element.
FIG. 1D is a cross sectional view showing the heat generation properties of a hypothetical modification to the FIG. 1B electrode element.
FIG. 2 is a plan schematic representation of an electrode assembly including electrode elements that is used for applying TTFields to a subject's body.
FIG. 3A is a cross sectional representation of a first embodiment including electrode elements E1, E2, taken along the dashed line in FIG. 2.
FIG. 3B is a cross sectional view showing the heat generation properties of the FIG. 3A embodiment.
FIG. 4A is a thermal image of a prior art electrode assembly.
FIG. 4B is a thermal image of an electrode assembly corresponding to the FIG. 3A embodiment.
FIG. 4C is a graph comparing the thermal properties of the prior art electrode assembly with the FIG. 3A embodiment.
FIG. 4D shows a thermal camera image of simulated electrodes arrays built using metal (aluminum) sheets.
FIG. 4E shows a thermal camera image of simulated electrodes arrays built using sheets of pyrolytic graphite.
FIG. 4F depicts experimental results when electrode arrays with and without a sheet of graphite were used to apply TTFields to the torso of rats.
FIG. 5 is a cross sectional representation of a second embodiment including electrode elements E1, E2, taken along the dashed line in FIG. 2.
FIG. 6 is a cross sectional representation of a third embodiment that includes a single electrode element E1.
FIG. 7 is a cross sectional representation of a fourth embodiment that includes a single electrode element E1.
FIG. 8 is a cross sectional representation of a fifth embodiment that includes a single electrode element E1.
FIG. 9 is a block diagram of a system incorporating two electrode assemblies that is used for applying TTFields to a subject's body.

Various embodiments are described in detail below with reference to the accompanying drawings, wherein like reference numerals represent like elements.

### DESCRIPTION OF THE PREFERRED EMBODIMENTS

This application describes exemplary electrode assemblies that may be used, e.g., for delivering TTFields to a subject's body and treating one or more cancers or tumors located in the subject's body.

When TTFields are applied to a subject's body, the temperature at the subject's body may increase proportionally to the induced electric field. Regulations limit the amount of current that can be driven through a transducer array to an amount that keeps the measured temperature at locations on the subject's body below a temperature threshold. As practiced in the art, the temperature at the location of the transducer arrays on the subject's body is controlled to be below the temperature threshold by reducing the operational current driven by the transducer arrays and reducing the strength of the resulting TTFields. This in turn becomes an over-riding limitation on the TTFields strength that can be used to treat the tumor. Accordingly, there is a need in the art to safely access higher TTField strengths without exceeding the temperature threshold at the subject's skin.

On transducer arrays that comprise multiple electrode elements, the portions of the transducer arrays positioned directly beneath the electrode elements get hotter than the portions of the transducer arrays positioned between the electrode elements. Furthermore, on transducer arrays that comprise multiple electrode elements, higher currents flow through the electrode elements located along the edge of the array compared to the electrode elements located toward the middle of the array. Further still, an electrode element located at a corner or similar sharp bend in the edge of the array will have a higher current than other electrode elements along the edge and near the center of the array. The tendency of a transducer array to drive higher currents through electrode elements located along the edge of the array, and particularly at the corners, is referred to herein as the "edge effect."

An uneven distribution of current through the transducer array due to either the distribution of the electrode elements or the edge effect can lead to higher temperature zones (or "hot spots") e.g., at the corners or edges of the transducer array. These hot spots are the locations that reach the threshold temperature first and therefore control the requirement to reduce the current. As such, the generation of hot spots limits the maximum operational current that may be driven by a transducer array, and the strength of the resulting TTFields.

The inventors have now recognized that a need exists for transducer arrays that reduce or minimize uneven distribution of current and thereby allow the application of higher operating currents. Transducer arrays operated with increased current can induce stronger TTFields in the subject's body, ultimately leading to better patient outcomes. The electrode assemblies disclosed herein allow current and heat to be spread evenly over the array thereby minimizing or eliminating hot spots.

The embodiments described herein incorporate a sheet of graphite into the electrode assembly, as described below. This lowers the temperature of the hot spots and raises the temperature of the cooler regions when a given AC voltage is applied to the electrode assembly (as compared to the prior art configuration described above). Accordingly, the current can be increased (thereby increasing the therapeutic effect) without exceeding the safety temperature threshold at any point on the subject's skin.

In some preferred embodiments, the sheet of graphite is a sheet of pyrolytic graphite. Notably, because graphite is nonmetallic, it advantageously prevents the transfer of ions into a subject's body.

The present invention can be understood more readily by reference to the following detailed description, examples, drawings, and claims, and their previous and following description. However, it is to be understood that this invention is not limited to the specific apparatuses, devices, systems, and/or methods disclosed unless otherwise specified, and as such, of course, can vary.

Headings are provided for convenience only and are not to be construed to limit the invention in any manner. Embodiments illustrated under any heading or in any portion of the disclosure may be combined with embodiments illustrated under the same or any other heading or other portion of the disclosure.

Any combination of the elements described herein and all possible variations thereof is encompassed by the invention unless otherwise indicated herein or otherwise clearly contradicted by context.

As used in the specification and the appended claims, the singular forms "a," "an" and "the" include plural referents unless the context clearly dictates otherwise.

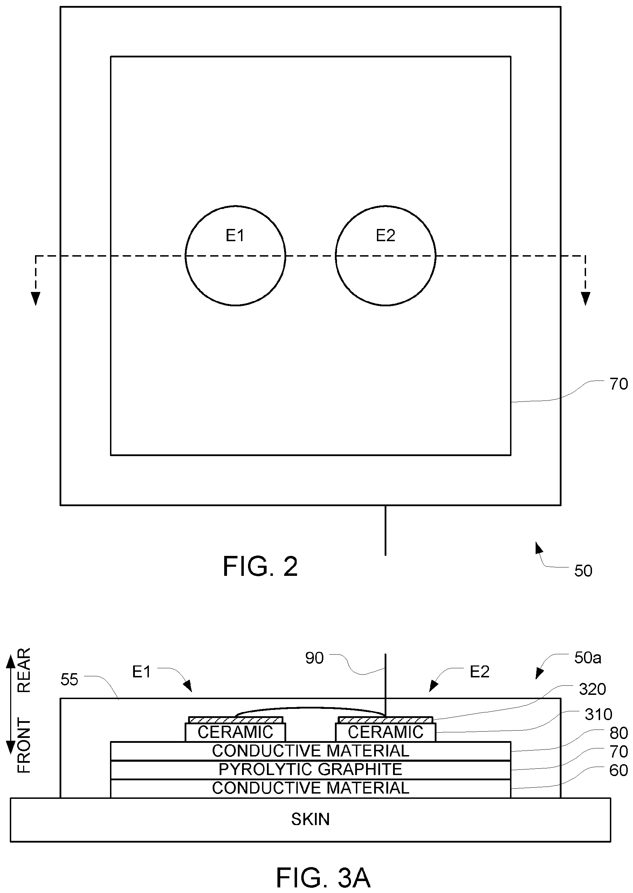

FIG. 2 is a schematic representation of an electrode assembly 50 of an embodiment including electrode elements used for applying TTFields to a subject's body. In FIG. 2, only two electrode elements labeled E1 and E2 are shown, but additional electrode elements may be included in the electrode assembly 50. In alternative embodiments, the electrode assembly 50 includes only a single electrode element. Notably, FIG. 2 depicts an electrode assembly 50 generically, and those electrode assemblies E1 and E2 can have different configurations (e.g., as described below in connection with FIGS. 3A-8).

FIG. 3A is a cross sectional representation of a first embodiment of an electrode assembly 50a including electrode elements E1, E2, taken along the dashed line in FIG. 2.

In the FIG. 3A embodiment, the electrode assembly 50a includes a sheet of pyrolytic graphite 70 having a front face (facing towards the subject's skin in FIG. 3A) and a rear face. Examples of suitable forms of graphite include synthetic graphite, such as pyrolytic graphite (including, but not limited to, Pyrolytic Graphite Sheet (PGS), available from Panasonic Industry, Kadoma, Osaka, Japan), other forms of synthetic graphite, including but not limited to, graphite foil made from compressed high purity exfoliated mineral graphite (including, but not limited to, that supplied by MinGraph^{®} 2010A Flexible Graphite, available from Mineral Seal Corp., Tucson, Arizona, USA), or graphitized polymer film, e.g., graphitized polyimide film, (including, but not limited to, that supplied by Kaneka Corp., Moka, Tochigi, Japan.

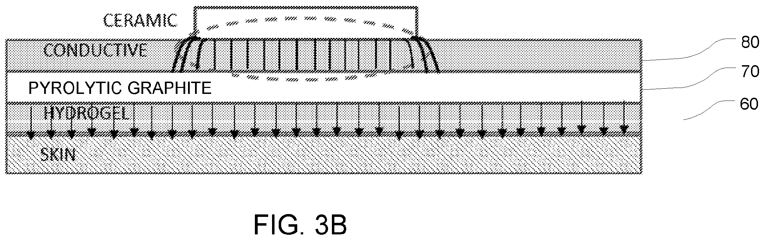

The electrode assembly 50a further includes at least one layer of conductive material 60 disposed on the front face of the sheet 70, and the at least one layer of conductive material 60 has a biocompatible front surface. Note that the embodiment illustrated in FIG. 3, there is only a single layer of conductive material 60, and that single layer is biocompatible. But in alternative embodiments (not shown) there could be more than one layer, in which case only the front layer must be biocompatible. The at least one layer of material 60 is configured to ensure good electrical contact between the device and the body. In some embodiments, the at least one layer of material 60 should cover the entire front face of the sheet of pyrolytic graphite 70. The at least one layer of material 60 may be the same size or larger than the sheet of pyrolytic graphite 70. In some embodiments (and as shown in FIG. 3A), the at least one layer of conductive material 60 comprises a single layer of hydrogel. In these embodiments, the hydrogel may have a thickness between 50 and 2000 µm, such as, from 100 to 1000 µm, or even 300 to 500 µm. In some embodiments, the at least one layer of conductive material 60 is a single layer of non-hydrogel biocompatible conductive adhesive. In some embodiments, the at least one layer of conductive material 60 is a single layer of non-hydrogel biocompatible conductive adhesive such as the developmental product FLX068983 - FLEXcon^{®} OMNI-WAVE^{™} TT 200 BLACK H-502 150 POLY H-9 44PP-8 from FLEXcon, Spencer, MA, USA, or other such OMNI-WAVE products from FLEXcon; or ARcare^{®} 8006 electrically conductive adhesive composition manufactured and sold by Adhesives Research, Inc. (Glen Rock, PA, USA). Non-hydrogel conductive adhesives may comprise a waterless polymer with adhesive properties and carbon particles, powder, fibers, flakes or nanotubes. The adhesive polymer may be, for example, an acrylic polymer or a silicone polymer, or combination thereof, which may be available as acrylic- or silicone-based carbon-filled adhesive tapes. The adhesive may additionally include one or more conductive polymer (such as, for example, polyaniline (PANI) or poly(3,4-ethylenedioxythiophene (PEDOT), or others known in the art). The conductive filler in the at least one layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50a further includes a first electrode element E1 positioned behind the sheet 70. The first electrode element E1 has a first front face disposed in electrical contact with the rear face of the sheet 70. In the FIG. 3A embodiment, the first electrode element E1 includes a first layer of dielectric (e.g., ceramic) material 310 having a front face and a rear face, and a first layer of metal 320 disposed on the rear face of the first layer of dielectric material 310. The front face of the first layer of dielectric material 310 is the first front face of the first electrode element E1. Note that while the figures (e.g., FIG. 3A) depict the dielectric material 310 as "ceramic," a variety of other suitable dielectric materials may be used instead of ceramic materials. Examples include a polymer layer that has a dielectric constant of at least 10, or another material having a dielectric constant of at least 10.

In some embodiments, the layer of dielectric material 310 can have a dielectric constant ranging from 10 to 50,000. In some embodiments, the layer of dielectric material 310 comprises a high dielectric polymer material such as poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF-TrFE-CTFE)" and "Poly(VDF-TrFE-CFE)," respectively. These embodiments are particularly advantageous because the dielectric constant of these materials is on the order of 40. In some embodiments, the polymer layer can be poly(vinylidene fluoride-trifluoroethylene-chlorotrifluoroethylene-chlorofluoroethylene) or "Poly(VDF-TrFE-CTFE-CFE)."

In some embodiments, the layer of dielectric material 310 comprises a terpolymer comprising polymerized units of monomers such as VDF, TrFE, CFE and/or CTFE in any suitable molar ratio. Suitable terpolymers include those, for example, having 30 to 80 mol% VDF, 5 to 60 mol% TrFE, with CFE and/or CTFE constituting the balance of the mol% of the terpolymer.

In some embodiments, the sheet 70 has a centroid, and the centroid of the first front face of the first electrode element E1 is positioned less than 3 cm away from the centroid of the sheet 70. In some embodiments, the sheet 70 has a centroid and a dimension parallel to the rear face of the sheet 70 (e.g., a length or a width), and the centroid of the first front face of the first electrode element E1 is positioned away from the centroid of the sheet 70 by less than 30%, or by less than 10% of the dimension.

The electrode assembly 50a further includes a first rear layer of conductive material 80 positioned between the first front face of the first electrode element E1 (i.e., the front face of the first layer of dielectric material 310) and the rear face of the sheet 70. The first rear layer of conductive material 80 facilitates the electrical contact between the first front face of the first electrode element E1 and the rear face of the sheet 70. In some embodiments, the rear layer of conductive material 80 is a layer of hydrogel. But in alternative embodiments, a different conductive material (e.g., conductive grease, conductive adhesive, conductive tape, conductive composite, etc.) could be used. In some embodiments, the conductive material 80 may be a non-hydrogel conductive adhesive, such as described above.

The electrode assembly 50a may optionally include one or more additional electrode elements. In the illustrated embodiment, the electrode assembly 50a includes a second electrode element E2 positioned behind the sheet 70. The second electrode element E2 has a second front face disposed in electrical contact with the rear face of the sheet 70. The two electrode elements E1, E2 in FIG. 3A have identical structures. Thus, the second electrode element E2 includes a second layer of dielectric (e.g., ceramic) material 310 having a front face and a rear face, and a second layer of metal 320 disposed on the rear face of the second layer of dielectric material 310. The front face of the second layer of dielectric material 310 is the second front face of the second electrode element E2. In some embodiments, the collective area of all the electrode elements is less than the area of the sheet 70, less than half the area of the sheet 70, less than one quarter the area of the sheet 70, or less than one tenth the area of the sheet 70.

The first rear layer of conductive material 80 is positioned between the second front face of the second electrode element E2 (i.e., the front face of the second layer of dielectric material 310) and the rear face of the sheet 70. The first rear layer of conductive material 80 facilitates the electrical contact between the second front face of the second electrode element E2 and the rear face of the sheet 70. As described for E1, and as shown in FIG. 3A, the conductive material 80 may be a layer of hydrogel, but in alternative embodiments, a different conductive material may be used (e.g., conductive grease, conductive adhesive including the non-hydrogel conductive adhesive described above, conductive tape, conductive composite, etc.).

The metal layers 320 of all of the electrode elements (i.e., E1 and E2 in the illustrated embodiment), may be wired together (e.g., using wires, traces on a flex circuit, etc.) to a lead 90. The lead 90 supplies an AC voltage from an AC voltage generator (not shown) to the electrode elements to generate the TTFields when the electrode assembly 50a is affixed to the subject's body for treatment.

Optionally, the electrode assembly 50a includes a flexible self-adhesive backing 55 configured to support the sheet 70, the first electrode element E1 (and any other electrode elements present in the electrode assembly), and the at least one layer of conductive material 60 so that the at least one layer of conductive material 60 can be positioned against the subject's skin.

As noted above, FIG. 2 is a plan schematic representation of an electrode assembly 50 including electrode elements E1, E2. This view of FIG. 2 (not to scale) also demonstrates that the area of the sheet 70 is larger (e.g., at least 2 times larger, at least 4 times larger, or at least 10 times larger) than the combined areas of the electrode elements E1, E2. When an AC voltage is applied to the electrode elements E1, E2, heat spreads out across the entire sheet 70, which minimizes or eliminates hot spots.

This reduction in hot spots (as compared to the prior art) becomes apparent by comparing FIG. 1C to FIG. 3B. More specifically, FIG. 1C shows the current distribution and heat generation for prior art electrode elements, each of which is positioned on a conductive hydrogel layer that covers about the same area as the electrode element. As shown in FIG. 1C, all the current passes through the hydrogel layer directly beneath the electrode elements, which results in hot spots directly beneath the electrode elements.

One might initially think that this problem could be solved by increasing the area of the hydrogel to cover all the regions between the electrode elements (i.e., by covering a significantly larger area in the x-y plane than that of the electrode elements). But this is not the case. More specifically, FIG. 1D shows the current distribution and heat generation for this hypothetical electrode assembly. As shown in FIG. 1D, all the current still passes through the hydrogel layer directly beneath the electrode elements, which results in hot spots directly beneath the electrode elements.

In contrast, FIG. 3B shows the current distribution for the FIG. 3A embodiment. As shown in FIG. 3B, the current is still distributed in the rear conductive material layer (for example, 80 in FIG. 3B) only in the area below the electrode element. However, the sheet of pyrolytic graphite 70 spreads the heat out across its entire area because the thermal conductivity in the horizontal directions is high. In addition to spreading out the heat, the low electrical resistance of the sheet 70 in the horizontal direction spreads the current outward throughout the sheet 70, and this spread-out current distribution continues in the layer of conductive material 60, and thence to the subject's skin. Because the current and heat in this embodiment are both spread out over a larger area of the layer of conductive material 60, hotspots are eliminated (or at least minimized). This means that for a given applied AC voltage, the hottest point beneath the electrode assembly in the FIG. 3A/B embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 3A embodiment. And this increase in current will advantageously increase the efficacy of the TTFields treatment. Similar results can be achieved when the hydrogel is replaced with a conductive adhesive composite.

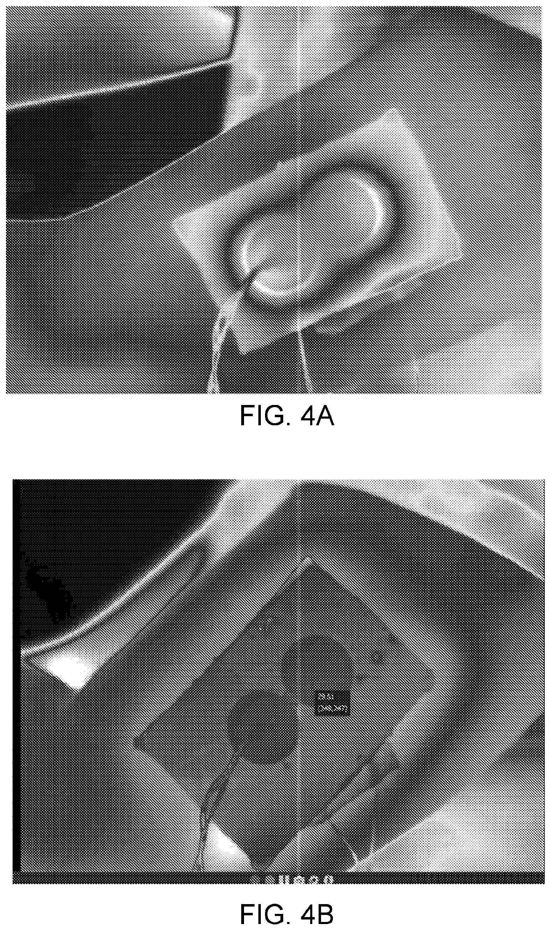

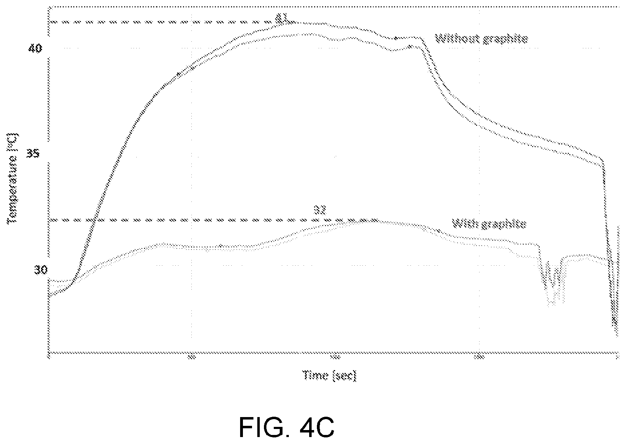

The superior performance of the FIG. 3A embodiment is demonstrated in FIGS. 4A, 4B, and 4C. FIG. 4A is a thermal image of a prior art electrode assembly that includes two electrode elements and a layer of hydrogel disposed on the front faces of the electrode elements. There is no sheet of graphite between the front faces of the electrode elements and the rear face of the layer of hydrogel. In use, the front face of the layer of hydrogel is positioned on the subject's skin. FIG. 4A shows hot spots generated in the areas that correspond to the electrode elements.

FIG. 4B is a thermal image of an electrode assembly corresponding to the FIG. 3A embodiment where pyrolytic graphite 70 is positioned between the front faces of the electrode elements E1, E2 and the rear face of the conductive layer 60, and the conductive layer 60 was made of hydrogel. FIG. 4B shows that hot spots such as those generated in the prior art electrode assembly have been minimized, and also that the maximum temperature has been reduced. FIG. 4C is a graph comparing the thermal performance of the FIG. 3A embodiment (with pyrolytic graphite) with the prior art (no graphite) for the same applied current (500 mA). Notably, the hottest portion of the prior art electrode assembly was 41° C. But when the same 500 mA current was applied to the FIG. 3A embodiment, the hottest portion of the electrode assembly was only 32° C. Similar experiments were performed utilizing graphite foil made from compressed high purity exfoliated mineral graphite, with similar results.

In a related experiment, optimized conventional arrays (no graphite sheet), running with 2 A applied current, ran up to the maximum 40 °C average temperature, and were thereby limited. The same type of array with an added pyrolytic graphite sheet (in the manner of the FIG. 3A embodiment) was able to run at an increased power level (with 3 A applied current), and ran at 38 °C average temperature, 2-3 °C below the temperature threshold limit. This result suggests that the inventive apparatus and methods described herein should be able to achieve more beneficial treatment results by operating at higher applied currents.

An experimental simulation of electrodes for treating a target location in a body compared the heat distribution obtained using graphite sheets to the heat distribution obtained using metal sheets. In one half of the experiment, a phantom gel was placed sandwiched between two sheets of metal (aluminum) and a voltage was applied (directly to the center of the sheet) between the two metal sheets. In the other half of the experiment, a phantom gel was placed sandwiched between two sheets of pyrolytic graphite and a voltage was applied (directly to the center of the sheet) between the two pyrolytic graphite sheets. When voltage is applied between a pair of metal sheets (aluminum), a higher current density at the edges of the sheet results in an unequal heating of different areas. In contrast, applying voltage between two graphite sheets beneficially results in much more uniform current densities at the sheet center and edges, and results in a more uniform temperature profile of the sheet.

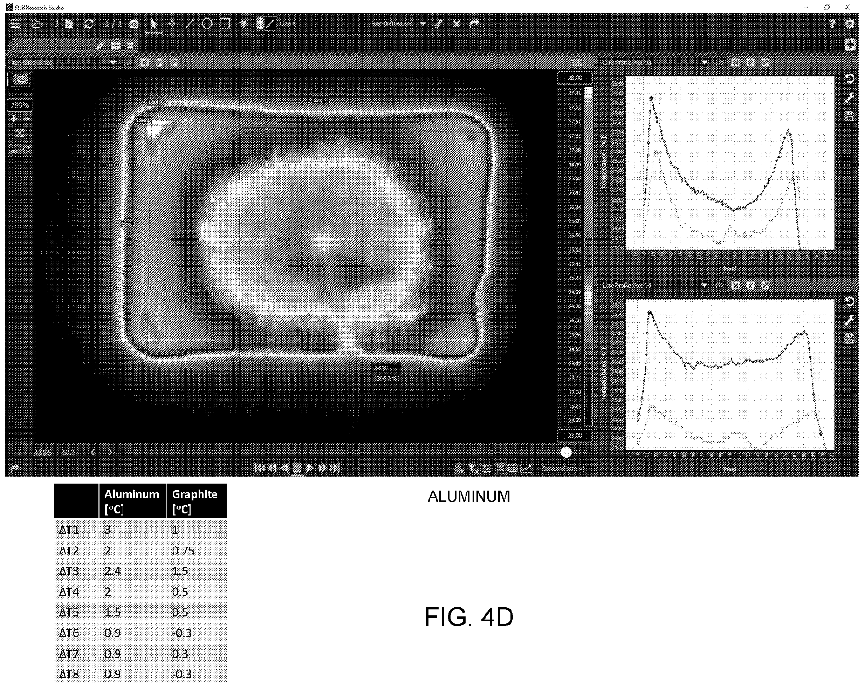

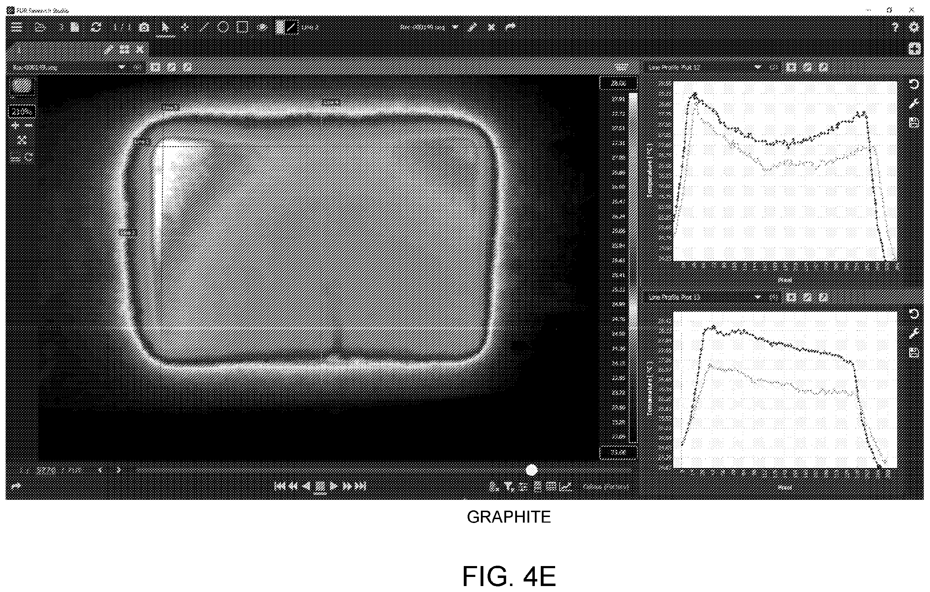

FIGS. 4D and 4E respectively show thermal camera images of the simulated electrodes arrays built using metal (aluminum) sheets and the simulated electrodes arrays built using sheets of pyrolytic graphite. The aluminum sheet results in an uneven heat distribution map which causes the outer edges to reach the threshold temperature first and therefore control the requirement to reduce the current. In contrast, the sheet of pyrolytic graphite produces a very even heat distribution across the entire sheet.

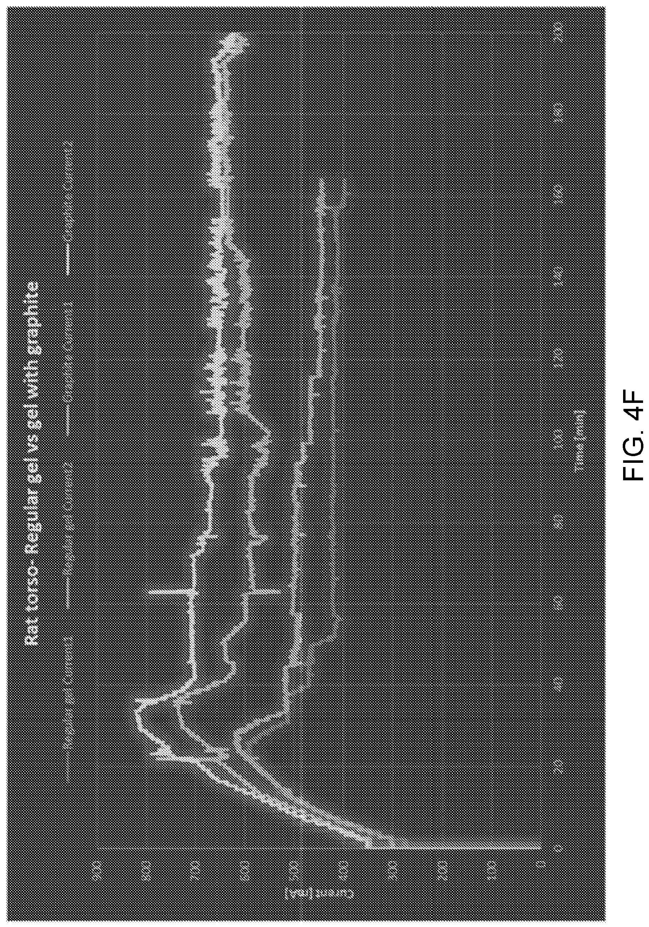

FIG. 4F depicts experimental results when electrode arrays with and without a sheet of graphite were used to apply TTFields to the torso of rats (using small animal arrays). The two lower traces show the measured current for two rats when the prior art electrode arrays depicted in FIG. 1A/1B were used, while the two upper traces show the measured current for two rats when the electrode elements depicted in FIG. 3A were used (using sheets of graphite). The thermal setpoint was identical for all runs. Notably, when the sheet of graphite was included, the improved heat and current distribution attributable to the graphite resulted in resistances that were 20% lower and currents that were 50% higher for the same thermal setpoint. And because higher currents are associated with improved outcomes, these experiments show that incorporating a layer of graphite into the electrode arrays can provide improved outcomes.

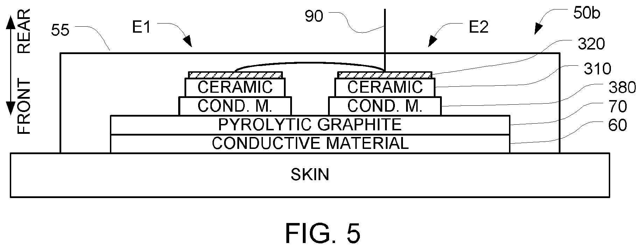

FIG. 5 is a cross sectional representation of a second embodiment of an electrode assembly 50b including electrode elements E1, E2, taken along the dashed line in FIG. 2. The FIG. 5 embodiment is similar to the FIG. 3A embodiment in all respects (including the figure labeling) except as follows. The FIG. 3A embodiment includes a large rear layer of conductive material 80 (e.g., hydrogel) positioned between the sheet 70 and the front faces of both the first and second electrode elements E1 and E2. In contrast, the FIG. 5 embodiment includes a separate region of conductive material 380 for each individual electrode element. Thus, the FIG. 5 embodiment includes a first rear layer of conductive material 380 positioned between the first front face of the first electrode element E1 and the rear face of the sheet 70, and also includes a second rear layer of conductive material 380 positioned between the second front face of the second electrode element E2 and the rear face of the sheet 70. The first and second rear layers of conductive material 380 facilitate the electrical contact between the respective electrode front faces and the rear face of the sheet 70. In some embodiments, the rear layers of conductive material 380 are layers of hydrogel. But in alternative embodiments, different conductive materials (e.g., conductive grease, conductive adhesive including the non-hydrogel conductive adhesives discussed above, conductive tape, conductive composite, etc.) could be used. In some embodiments, the collective area of all the electrode elements is less than the area of the sheet 70, less than half the area of the sheet 70, less than one quarter the area of the sheet 70, or less than one tenth the area of the sheet 70.

As in the FIG. 3A embodiment, the current in the FIG. 5 embodiment is still concentrated in the rear layers of conductive material 380 only in the areas below the electrode elements. The sheet of pyrolytic graphite 70 spreads out the heat and the current as described above in connection with the FIG. 3A embodiment, which eliminates or at least minimizes hot spots. This means that for a given applied AC voltage, the hottest point beneath the electrode assembly in the FIG. 5 embodiment will be at a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 5 embodiment. And this increase in current will advantageously increase the efficacy of the TTFields treatment.

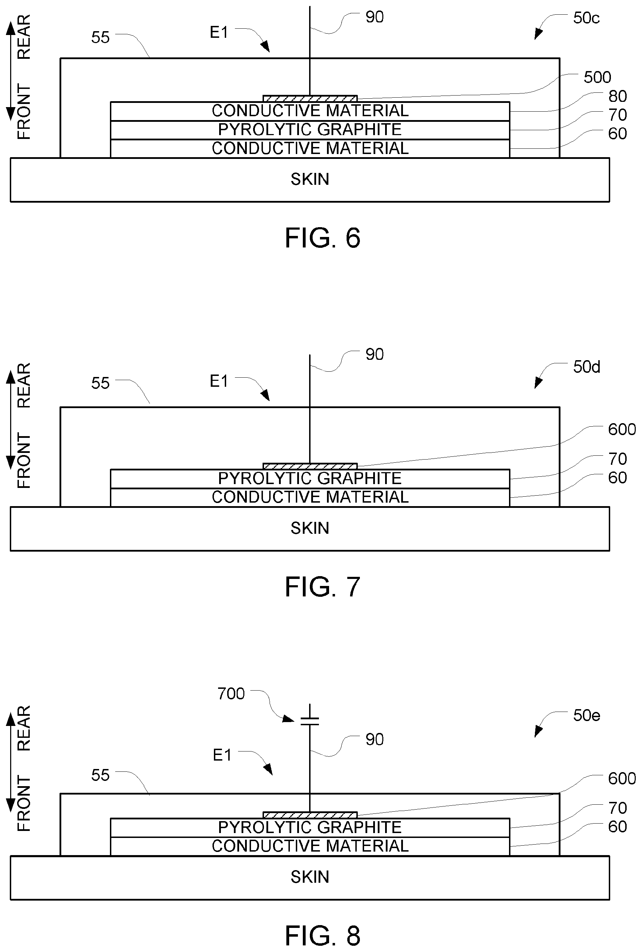

FIG. 6 is a cross sectional representation of a third embodiment of an electrode assembly 50c that includes a single electrode element E1. The embodiment of FIG. 6 is similar to the embodiment of FIG. 3A except the FIG. 6 embodiment does not include the layer of dielectric material. In the FIG. 6 embodiment, the electrode assembly 50c includes a sheet of pyrolytic graphite 70 having a front face (facing towards the subject's skin in FIG. 6) and a rear face. This sheet 70 is similar to the sheet 70 described above in connection with FIG. 3A.

The electrode assembly 50c further includes at least one layer of conductive material 60 disposed on the front face of the sheet 70, and the at least one layer of conductive material 60 has a biocompatible front surface. Note that the embodiment illustrated in FIG. 6, there is only a single layer of conductive material 60, and that single layer is biocompatible. But in alternative embodiments (not shown) there could be more than one layer, in which case only the front layer must be biocompatible. The at least one layer of conductive material 60 is configured to ensure good electrical contact between the device and the body. In a preferred embodiment, the at least one layer of conductive material 60 should cover the entire front face of the sheet of pyrolytic graphite 70. The at least one layer of conductive material 60 may be the same size or larger (i.e., cover the same area or larger) than the sheet of pyrolytic graphite 70. In some embodiments, the at least one layer of conductive material 60 comprises a single layer of hydrogel. In these embodiments, the hydrogel may have a thickness between 50 and 2000 µm, such as, from 100 to 1000 µm, or even 300 to 500 µm. In some embodiments, the at least one layer of conductive material 60 is a single layer of non-hydrogel biocompatible conductive adhesive as discussed above. In some embodiments, the at least one layer of conductive material 60 is a single layer of non-hydrogel biocompatible conductive adhesive such as the OMNI-WAVE products from FLEXcon or the ARcare^{®} products from Adhesives Research, Inc., discussed above. Non-hydrogel conductive adhesives may comprise a waterless polymer with adhesive properties (for example, an acrylic polymer or a silicone polymer, or combination thereof) and a conductive filler. The conductive filler in the at least one layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50c further includes a first electrode element E1 positioned behind the sheet 70. The first electrode element E1 includes a piece of metal 500 having a front face disposed in electrical contact with the rear face of the sheet 70. In the FIG. 6 embodiment, the front face of the piece of metal 500 is the first front face of the first electrode element E1. Accordingly, the FIG. 6 embodiment differs from the FIG. 3A or FIG. 5 embodiments by lacking a layer of dielectric material. The positional relationship between the first electrode element E1 and the sheet 70 in this FIG. 6 embodiment may be as described above in connection with FIG. 3A.

The electrode assembly 50c further includes a first rear layer of conductive material 80 positioned between the first front face of the first electrode element E1 (i.e., the front face of the piece of metal 500) and the rear face of the sheet 70. The first rear layer of conductive material 80 facilitates the electrical contact between the first front face of the first electrode element E1 and the rear face of the sheet 70. In some embodiments, the rear layer of conductive material 80 is a layer of hydrogel. But in alternative embodiments, a different conductive material (e.g., conductive grease, conductive adhesive including the non-hydrogel conductive adhesive described above, conductive tape, conductive composite, etc.) could be used.

The piece of metal 500 of the electrode element E1 is wired (e.g., using wires, traces on a flex circuit, etc.) to a lead 90, which supplies an AC voltage from an AC voltage generator (not shown) to the electrode element to generate the TTFields when the electrode assembly 50c is affixed to the subject's body for treatment.

The electrode assembly 50c may optionally include one or more additional electrode elements (not shown) having a structure identical to electrode element E1 and positioned to have the same functionality. In such case, the pieces of metal 500 of all the electrode elements may be wired together (e.g., using wires, traces on a flex circuit, etc.) to the lead 90.

In some embodiments that include only a single electrode element E1, the area of the sheet 70 is larger (e.g., at least 2 times larger, at least 4 times larger, or at least 10 times larger) than the area of the electrode element E1. In some embodiments that include a plurality of electrode elements (not shown) the area of the sheet 70 is larger (e.g., at least 2, 4, or 10 times larger) than the collective area of all of the electrode elements. When an AC voltage is applied to the electrode elements, the heat spreads out across the entire sheet 70, which minimizes or eliminates hot spots.

Similar to the FIG. 3A embodiment, the sheet of pyrolytic graphite 70 in the FIG. 6 embodiment spreads out the heat and the current as described above in connection with the FIG. 3A embodiment, which eliminates or at least minimizes hot spots. This means that for a given applied AC voltage, the hottest point beneath the electrode assembly in the FIG. 6 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 6 embodiment. And this increase in current will advantageously increase the efficacy of the TTFields treatment.

FIG. 7 is a cross sectional representation of a fourth embodiment of an electrode assembly 50d that includes a single electrode element E1. The FIG. 7 embodiment is similar to the FIG. 6 embodiment except that the first front face of the first electrode element E1 (i.e., the front face of the piece of metal 600) is positioned in direct contact with the rear face of the sheet 70 (instead of being electrically connected via an intervening layer of conductive material).

Similar to the FIG. 6 embodiment, the sheet of pyrolytic graphite 70 in the FIG. 7 embodiment spreads out the heat and the current as described above in connection with the FIG. 3A embodiment, which eliminates or at least minimizes hot spots. This means that for a given applied AC voltage, the hottest point beneath the electrode assembly in the FIG. 7 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 7 embodiment. And this increase in current will advantageously increase the efficacy of the TTFields treatment.

FIG. 8 is a cross sectional representation of a fifth embodiment of an electrode assembly 50e that includes a single electrode element E1. The FIG. 8 embodiment is similar to the FIG. 7 embodiment, but it adds a capacitor 700 connected in series with and behind the piece of metal 600. A similar addition of a capacitor 700 connected in series with and behind the piece of metal 600 could also be envisioned for the FIG. 6 embodiment.

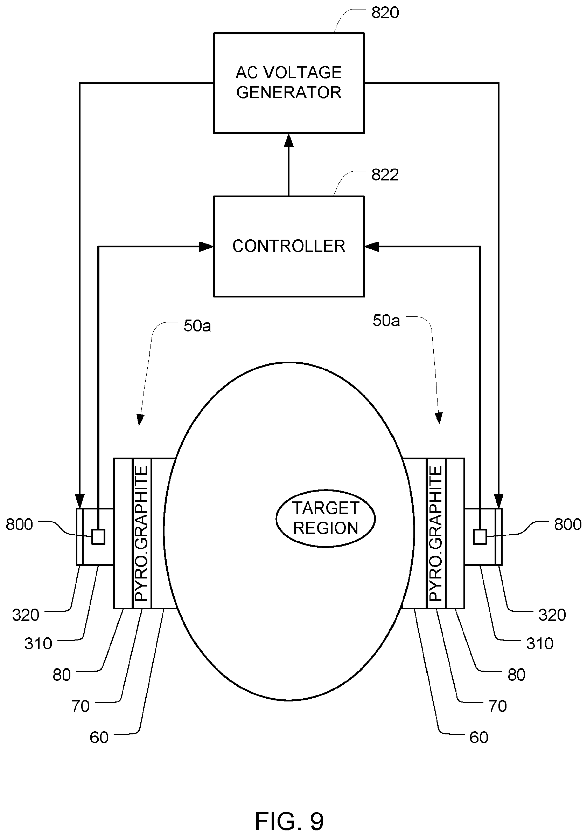

FIG. 9 shows how a pair of the FIG. 3A electrode assemblies 50a may be used to apply an alternating electric field to a target region in the subject's body. The subject could be a human or another mammal, including but not limited to rats and mice. (Note that any of the electrode assemblies described above in connection with FIGS. 5-8 may be used instead of the FIG. 3A electrode assemblies 50a shown here).

The method includes positioning a first electrode assembly 50a at a first position on or in the subject's body. (In the example depicted in FIG. 9, the first electrode assembly 50a is positioned on the subject's skin at the right of the subject's head facing a target region, e.g., a tumor.) The first electrode assembly 50a may be constructed as described earlier herein. In the FIG. 9 embodiment, the first electrode assembly 50a includes a first sheet 70 of pyrolytic graphite 70 having a first front face and a first rear face. During use, the first electrode assembly 50a is positioned so that the first front face of the first sheet 70 faces the target region.

The method also includes positioning a second electrode assembly 50a at a second position in or on the subject's body. (In the example depicted in FIG. 9, the second electrode assembly 50a is positioned on the subject's skin at the left of the subject's head facing the target region.) The second electrode assembly 50a may be constructed as described earlier herein. In the FIG. 9 embodiment, the second electrode assembly 50a includes a second sheet 70 of pyrolytic graphite 70 having a second front face and a second rear face. During use, the second electrode assembly 50a is positioned so that the second front face of the second sheet 70 faces the target region.

The method further includes applying an alternating voltage between the first electrode assembly 50a and the second electrode assembly 50a. The applying is performed after positioning the first electrode assembly 50a and the second electrode assembly 50a. The applying may be implemented by applying the alternating voltage between (i) a first electrode element disposed in electrical contact with the first rear face of the first sheet 70 and (ii) a second electrode element disposed in electrical contact with the second rear face of the second sheet 70.

In some embodiments, the first electrode assembly 50a further includes a first layer of biocompatible conductive material 60 disposed on the first front face of the first sheet 70. Correspondingly, the second electrode assembly further includes a second layer of biocompatible conductive material 60 disposed on the second front face of the second sheet 70. As described above, the biocompatible conductive material 60 may be hydrogel or may be a conductive grease, conductive adhesive including the non-hydrogel conductive adhesives discussed above, conductive tape, conductive composite, etc.

In some embodiments, the first electrode assembly 50a further includes a first rear layer of conductive material 80 (as described above) positioned between the first front face of the first electrode element of the first electrode assembly 50a and the first rear face of the first sheet 70. Correspondingly, the second electrode assembly further includes a second rear layer of conductive material 80 (as described above) positioned between the second front face of the second electrode element of the second electrode assembly and the second rear face of the second sheet 70.

The alternating voltage between the first electrode assembly and the second electrode assembly may be applied by an AC voltage generator 820. In some embodiments, the frequency of the alternating voltage is between 50 kHz and 1 MHz, or between 100 kHz and 500 kHz. In the illustrated example, the AC voltage generator is controlled by a controller 822. The controller 822 may use temperature measurements to control the amplitude of the current to be delivered via the first and second electrode assemblies 50a in order to maintain temperatures below a safety threshold (e.g., 41° C). This may be accomplished, for example, by measuring a first temperature of the first electrode element, measuring a second temperature of the second electrode element, and controlling the applying of the alternating voltage based on the first temperature and the second temperature, as described below.

FIG. 9 depicts one example of hardware that is suitable for this purpose. More specifically, temperature sensors 800 (e.g., thermistors) are positioned in thermal contact with respective electrode elements 310/320 within each of the electrode assemblies 50a. The temperature sensors 800 measure respective first and second temperatures (e.g., at first and second electrode elements in the first electrode assembly and second electrode assembly, respectively), and the controller 822 controls the output of the AC voltage generator 820 based on these temperatures.

Similar embodiments and methods are envisaged utilizing any of the electrode assemblies 50a-e, or combinations thereof, in place of either or both of the first electrode assembly 50a and the second electrode assembly 50a.

In the embodiments discussed above in connection with FIG. 2-9, the sheet 70 is made of pyrolytic graphite. But in alternative embodiments, the sheet 70 may be made of other types of graphite, including but not limited to other synthetic graphite, such as graphite foil made from compressed high purity exfoliated mineral graphite (including but not limited to MinGraph^{®} 2010A Flexible Graphite, available from Mineral Seal Corp., Tucson, Arizona, USA); isotropic graphite (including but not limited to isotropic graphite grade G330 available from Tokai Carbon Europe, Oldbury, UK; or double-sided carbon tape for scanning electron microscopy, available from Fisher Scientific, a unit of Thermo Fisher Scientific, Hampton, NH, USA).

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. An apparatus for applying an alternating electric field to a subject's body, the apparatus comprising:
a sheet of graphite (70) having a front face and a rear face, wherein the sheet of graphite (70) has a high thermal conductivity in a horizontal direction, parallel to the front face thereof, such that the sheet of graphite (70) is configured to spread heat out across the entire area of the sheet of graphite (70);
at least one layer of conductive material (60) disposed in electrical contact with the front face of the sheet of graphite (70), wherein the at least one layer of conductive material (60) has a biocompatible front surface; and
at least one electrode element (E1, E2) positioned behind the sheet of graphite (70), wherein the at least one electrode element (E1, E2) has a front face disposed in electrical contact with the rear face of the sheet of graphite (70).

2. The apparatus of claim 1, wherein the at least one electrode element (E1, E2) comprises a layer of dielectric material (310) having a front face and a rear face and a layer of metal (320) disposed on the rear face of the layer of dielectric material (310), the front face of the layer of dielectric material (310) being the front face of the at least one electrode element (E1, E2); and
further comprising:
a rear layer of conductive material (80) positioned between the front face of the at least one electrode element (E1, E2) and the rear face of the sheet of graphite (70), wherein the rear layer of conductive material (80) is configured to facilitate the electrical contact between the front face of the at least one electrode element (E1, E2) and the rear face of the sheet of graphite (70).

3. The apparatus of claim 1, wherein the at least one electrode element (E1, E2) comprises a layer of dielectric material (310) having a front face and a rear face, a layer of metal (320) disposed on the rear face of the layer of dielectric material (310), the front face of the layer of dielectric material (310) being the front face of the at least one electrode element (E1, E2), and a rear layer of conductive material (380) positioned between the front face of the at least one electrode element (E1, E2) and the rear face of the sheet of graphite (70), wherein the rear layer of conductive material (380) is configured to facilitate the electrical contact between the front face of the at least one electrode element (E1, E2) and the rear face of the sheet of graphite (70).

4. The apparatus of claim 2 or 3, wherein the rear layer of conductive material (80; 380) comprises conductive hydrogel.

5. The apparatus of claim 2 or 3, wherein the rear layer of conductive material (80; 380) comprises a conductive adhesive, optionally the conductive adhesive comprises an adhesive polymer and carbon powder, particles, fibers, flakes or nanotubes.

6. The apparatus of claim 4, wherein the conductive adhesive has a thickness between 10 and 2,000 µm.

7. The apparatus of claim 1, wherein the at least one electrode element (E1, E2) comprises a piece of metal (500; 600) having a front face, the front face of the piece of metal (500; 600) being the front face of the at least one electrode element (E1, E2), optionally the front face of the at least one electrode element (E1, E2) is positioned in direct contact with the rear face of the sheet of graphite (70).

8. The apparatus of claim 7, further comprising:
a rear layer of conductive material (80; 380) positioned between the front face of the at least one electrode element (E1, E2) and the rear face of the sheet of graphite (70), wherein the rear layer of conductive material (80; 380) is configured to facilitate the electrical contact between the front face of the at least one electrode element (E1, E2) and the rear face of the sheet of graphite (70).

9. The apparatus of claim 1, wherein the sheet of graphite (70) is a sheet of pyrolytic graphite.

10. The apparatus of claim 1, wherein the sheet of graphite (70) is a sheet of graphite foil of compressed high-purity exfoliated mineral graphite or a graphitized polymer film.

11. The apparatus of claim 1, wherein the at least one layer of conductive material (60) comprises hydrogel.

12. The apparatus of claim 1, wherein the at least one layer of conductive material (60) comprises a layer of hydrogel with a thickness between 50 and 2000 µm.

13. The apparatus of claim 1, wherein the at least one layer of conductive material (60) comprises a conductive adhesive, optionally the conductive adhesive comprises an adhesive polymer and carbon powder, particles, fibers, flakes or nanotubes.

14. The apparatus of claim 1, wherein the at least one layer of conductive material (60) comprises a conductive composite, optionally the conductive composite comprises an acrylic or silicone polymer and carbon powder, particles, fibers, flakes or nanotubes.

15. The apparatus of claim 1, further comprising:
a flexible self-adhesive backing (55) configured to support the sheet of graphite (70), the at least one electrode element (E1, E2) and the at least one layer of conductive material (60) so that the front surface of the at least one layer of conductive material (60) can be positioned against a subject's skin.

16. The apparatus of claim 1, comprising a plurality of electrode elements (E1, E2).

17. The apparatus of claim 1, wherein the at least one layer of conductive material (60) is the same size or larger than the sheet of graphite (70).

18. The apparatus of claim 1, wherein the sheet of graphite (70) has a low electrical resistance in the horizontal direction, such that the sheet of graphite (70) is configured to spread current outwards throughout the sheet of graphite (70).

## Patentansprüche

1. Einrichtung zum Anlegen eines elektrischen Wechselfeldes an einen Körper eines Subjekts, wobei die Einrichtung umfasst:
ein Blatt aus Graphit (70), die eine Vorderseite und eine Rückseite aufweist, wobei das Blatt aus Graphit (70) eine hohe Wärmeleitfähigkeit in horizontaler Richtung parallel zur Vorderseite davon aufweist, sodass das Blatt aus Graphit (70) konfiguriert ist, um Wärme über die gesamte Fläche des Blattes aus Graphit (70) auszubreiten;
mindestens eine Schicht aus leitfähigem Material (60), die in elektrischem Kontakt mit der Vorderseite des Blattes aus Graphit (70) angeordnet ist, wobei die mindestens eine Schicht aus leitfähigem Material (60) eine biokompatible Vorderseite aufweist; und
mindestens ein Elektrodenelement (E1, E2), das hinter dem Blatt aus Graphit (70) positioniert ist, wobei das mindestens eine Elektrodenelement (E1, E2) eine Vorderseite aufweist, die in elektrischem Kontakt mit der Rückseite des Blattes aus Graphit (70) angeordnet ist.

2. Einrichtung nach Anspruch 1, wobei das mindestens eine Elektrodenelement (E1, E2) eine Schicht aus dielektrischem Material (310) umfasst, die eine Vorderseite und eine Rückseite und eine Metallschicht (320) aufweist, die auf der Rückseite der Schicht aus dielektrischem Material (310) angeordnet ist, wobei die Vorderseite der Schicht aus dielektrischem Material (310) die Vorderseite des mindestens einen Elektrodenelements (E1, E2) ist; und
weiter umfassend:
eine rückseitige Schicht aus leitfähigem Material (80), die zwischen der Vorderseite des mindestens einen Elektrodenelements (E1, E2) und der Rückseite des Blattes aus Graphit (70) positioniert ist, wobei die rückseitige Schicht aus leitfähigem Material (80) konfiguriert ist, um den elektrischen Kontakt zwischen der Vorderseite des mindestens einen Elektrodenelements (E1, E2) und der Rückseite des Blattes aus Graphit (70) zu erleichtern.

3. Einrichtung nach Anspruch 1, wobei das mindestens eine Elektrodenelement (E1, E2) eine Schicht aus dielektrischem Material (310) umfasst, die eine Vorderseite und eine Rückseite aufweist, eine Metallschicht (320), die auf der Rückseite der Schicht aus dielektrischem Material (310) angeordnet ist, wobei die Vorderseite der Schicht aus dielektrischem Material (310) die Vorderseite des mindestens einen Elektrodenelements (E1, E2) ist, und eine rückseitige Schicht aus leitfähigem Material (380), die zwischen der Vorderseite des mindestens einen Elektrodenelements (E1, E2) und der Rückseite des Blattes aus Graphit (70) positioniert ist, wobei die rückseitige Schicht aus leitfähigem Material (380) konfiguriert ist, um den elektrischen Kontakt die zwischen der Vorderseite des mindestens einen Elektrodenelements (E1, E2) und der Rückseite des Blattes aus Graphit (70) zu erleichtern.

4. Einrichtung nach Anspruch 2 oder 3, wobei die rückseitige Schicht aus leitfähigem Material (80; 380) leitfähiges Hydrogel umfasst.

5. Einrichtung nach Anspruch 2 oder 3, wobei die rückseitige Schicht aus leitfähigem Material (80; 380) einen leitfähigen Klebstoff umfasst, wobei der leitfähige Klebstoff optional ein Klebstoffpolymer und Kohlenstoffpulver, -partikel, -fasern, -flocken oder - nanoröhren umfasst.

6. Einrichtung nach Anspruch 4, wobei der leitfähige Klebstoff eine Dichte zwischen 10 und 2.000 µm aufweist.

7. Einrichtung nach Anspruch 1, wobei das mindestens eine Elektrodenelement (E1, E2) ein Metallstück (500; 600) umfasst, das eine Vorderseite aufweist, wobei die Vorderseite des Metallstücks (500; 600) die Vorderseite des mindestens einen Elektrodenelements (E1, E2) ist, wobei die Vorderseite des mindestens einen Elektrodenelements (E1, E2) optional in direktem Kontakt mit der Rückseite des Blattes aus Graphit (70) positioniert ist.

8. Einrichtung nach Anspruch 7, weiter umfassend:
eine rückseitige Schicht aus leitfähigem Material (80; 380), die zwischen der Vorderseite des mindestens einen Elektrodenelements (E1, E2) und der Rückseite des Blattes aus Graphit (70) positioniert ist, wobei die rückseitige Schicht aus leitfähigem Material (80; 380) konfiguriert ist, um den elektrischen Kontakt zwischen der Vorderseite des mindestens einen Elektrodenelements (E1, E2) und der Rückseite des Blattes aus Graphit (70) zu erleichtern.

9. Einrichtung nach Anspruch 1, wobei das Blatt aus Graphit (70) ein Blatt aus pyrolytischem Graphit ist.

10. Einrichtung nach Anspruch 1, wobei das Blatt aus Graphit (70) ein Blatt aus Graphitfolie aus komprimiertem, hochreinem, exfoliertem Mineralgraphit oder ein graphitisierter Polymerfilm ist.

11. Einrichtung nach Anspruch 1, wobei die mindestens eine Schicht aus leitfähigem Material (60) Hydrogel umfasst.

12. Einrichtung nach Anspruch 1, wobei die mindestens eine Schicht aus leitfähigem Material (60) eine Hydrogelschicht mit einer Dichte zwischen 50 und 2.000 µm umfasst.

13. Einrichtung nach Anspruch 1, wobei die mindestens eine Schicht aus leitfähigem Material (60) einen leitfähigen Klebstoff umfasst, wobei der leitfähige Klebstoff optional ein Klebstoffpolymer und Kohlenstoffpulver, -partikel, -fasern, -flocken oder -nanoröhren umfasst.

14. Einrichtung nach Anspruch 1, wobei die mindestens eine Schicht aus leitfähigem Material (60) einen leitfähigen Verbundstoff umfasst, wobei der leitfähige Verbundstoff optional ein Acryl- oder Silikonpolymer und Kohlenstoffpulver, -partikel, -fasern, -flocken oder -nanoröhren umfasst.

15. Einrichtung nach Anspruch 1, weiter umfassend:
eine flexible, selbstklebende Unterlage (55), die konfiguriert ist, um das Blatt aus Graphit (70), das mindestens eine Elektrodenelement (E1, E2) und die mindestens eine Schicht aus leitfähigem Material (60) zu tragen, sodass die vordere Oberfläche der mindestens einen Schicht aus leitfähigem Material (60) an der Haut eines Subjekts positioniert werden kann.

16. Einrichtung nach Anspruch 1, umfassend eine Vielzahl von Elektrodenelementen (E1, E2).

17. Einrichtung nach Anspruch 1, wobei die mindestens eine Schicht aus leitfähigem Material (60) gleich groß wie oder größer als das Blatt aus Graphit (70) ist.

18. Einrichtung nach Anspruch 1, wobei die das Blatt aus Graphit (70) einen niedrigen elektrischen Widerstand in horizontaler Richtung aufweist, sodass das Blatt aus Graphit (70) konfiguriert ist, um Strom nach außen durch das Blatt aus Graphit (70) hindurch auszubreiten.

## Revendications

1. Appareil permettant d'appliquer un champ électrique alternatif au corps d'un patient, l'appareil comprenant :
une feuille de graphite (70) présentant une face avant et une face arrière, dans lequel la feuille de graphite (70) présente une forte conductivité thermique dans une direction horizontale, parallèle à sa face avant, de sorte que la feuille de graphite (70) est configurée pour diffuser de la chaleur sur toute la surface de la feuille de graphite (70) ;
au moins une couche de matériau conducteur (60) disposée en contact électrique avec la face avant de la feuille de graphite (70), dans lequel la au moins une couche de matériau conducteur (60) présente une surface avant biocompatible ; et
au moins un élément d'électrode (E1, E2) positionné derrière la feuille de graphite (70), dans lequel le au moins un élément d'électrode (E1, E2) présente une face avant disposée en contact électrique avec la face arrière de la feuille de graphite (70).

2. Appareil selon la revendication 1, dans lequel le au moins un élément d'électrode (E1, E2) comprend une couche de matériau diélectrique (310) présentant une face avant et une face arrière et une couche de métal (320) disposée sur la face arrière de la couche de matériau diélectrique (310), la face avant de la couche de matériau diélectrique (310) étant la face avant du au moins un élément d'électrode (E1, E2) ; et
comprenant en outre :
une couche arrière de matériau conducteur (80) positionnée entre la face avant du au moins un élément d'électrode (E1, E2) et la face arrière de la feuille de graphite (70), dans lequel la couche arrière de matériau conducteur (80) est configurée pour faciliter le contact électrique entre la face avant du au moins un élément d'électrode (E1, E2) et la face arrière de la feuille de graphite (70).

3. Appareil selon la revendication 1, dans lequel le au moins un élément d'électrode (E1, E2) comprend une couche de matériau diélectrique (310) présentant une face avant et une face arrière, une couche de métal (320) disposée sur la face arrière de la couche de matériau diélectrique (310), la face avant de la couche de matériau diélectrique (310) étant la face avant du au moins un élément d'électrode (E1, E2), et une couche arrière de matériau conducteur (380) positionnée entre la face avant du au moins un élément d'électrode (E1, E2) et la face arrière de la feuille de graphite (70), dans lequel la couche arrière de matériau conducteur (380) est configurée pour faciliter le contact électrique entre la face avant du au moins un élément d'électrode (E1, E2) et la face arrière de la feuille de graphite (70).

4. Appareil selon la revendication 2 ou 3, dans lequel la couche arrière de matériau conducteur (80 ; 380) comprend un hydrogel conducteur.

5. Appareil selon la revendication 2 ou 3, dans lequel la couche arrière de matériau conducteur (80 ; 380) comprend un adhésif conducteur, facultativement l'adhésif conducteur comprend un polymère adhésif et de la poudre, des particules, des fibres, des flocons ou des nanotubes de carbone.

6. Appareil selon la revendication 4, dans lequel l'adhésif conducteur présente une épaisseur comprise entre 10 et 2 000 µm.

7. Appareil selon la revendication 1, dans lequel le au moins un élément d'électrode (E1, E2) comprend une pièce de métal (500 ; 600) présentant une face avant, la face avant de la pièce de métal (500 ; 600) étant la face avant du au moins un élément d'électrode (E1, E2), facultativement la face avant du au moins un élément d'électrode (E1, E2) est positionnée en contact direct avec la face arrière de la feuille de graphite (70).

8. Appareil selon la revendication 7, comprenant en outre :
une couche arrière de matériau conducteur (80 ; 380) positionnée entre la face avant du au moins un élément d'électrode (E1, E2) et la face arrière de la feuille de graphite (70), dans lequel la couche arrière de matériau conducteur (80 ; 380) est configurée pour faciliter le contact électrique entre la face avant du au moins un élément d'électrode (E1, E2) et la face arrière de la feuille de graphite (70).

9. Appareil selon la revendication 1, dans lequel la feuille de graphite (70) est une feuille de graphite pyrolytique.

10. Appareil selon la revendication 1, dans lequel la feuille de graphite (70) est une feuille de graphite en graphite minéral exfolié compressé de grande pureté ou un film polymère graphitisé.

11. Appareil selon la revendication 1, dans lequel la au moins une couche de matériau conducteur (60) comprend un hydrogel.

12. Appareil selon la revendication 1, dans lequel la au moins une couche de matériau conducteur (60) comprend une couche d'hydrogel d'une épaisseur comprise entre 50 et 2 000 µm.

13. Appareil selon la revendication 1, dans lequel la au moins une couche de matériau conducteur (60) comprend un adhésif conducteur, facultativement l'adhésif conducteur comprend un polymère adhésif et de la poudre, des particules, des fibres, des flocons ou des nanotubes de carbone.

14. Appareil selon la revendication 1, dans lequel la au moins une couche de matériau conducteur (60) comprend un composite conducteur, facultativement le composite conducteur comprend un polymère acrylique ou de silicone et de la poudre, des particules, des fibres, des flocons ou des nanotubes de carbone.

15. Appareil selon la revendication 1, comprenant en outre :
un support auto-adhésif flexible (55) configuré pour supporter la feuille de graphite (70), le au moins un élément d'électrode (E1, E2) et la au moins une couche de matériau conducteur (60) de telle sorte que la surface avant de la au moins une couche de matériau conducteur (60) peut être positionnée contre la peau d'un patient.

16. Appareil selon la revendication 1, comprenant une pluralité d'éléments d'électrode (E1, E2).

17. Appareil selon la revendication 1, dans lequel la au moins une couche de matériau conducteur (60) est d'une taille identique ou supérieure à la feuille de graphite (70).

18. Appareil selon la revendication 1, dans lequel la feuille de graphite (70) présente une faible résistance électrique dans la direction horizontale, de sorte que la feuille de graphite (70) est configurée pour diffuser un courant vers l'extérieur dans l'ensemble de la feuille de graphite (70).
